# EUROPEAN PATENT APPLICATION

(11) **EP 4 606 349 A1**
(43) Date of publication of application: **27.08.2025**
(21) Application number: 25709034.0
(22) Date of filing: 09.01.2025
(51) Int. Cl.: A61F 2/14, A61F 9/00

(54) **EYE DISEASE IMPLANT INSERTION DEVICE CAPABLE OF FIXING STENT**

(30) Priority: 09.01.2024 KR 20240003771
(71) Applicant: Microt Inc., Seoul 06351 (KR)
(72) Inventor: KIM, Seongjun, Seoul 04759 (KR); LEE, Jun Keun, Seoul 02157 (KR)
(74) Representative: Botti & Ferrari S.p.A.
(86) International application number: PCT/KR2025/000510
(87) International publication number: WO 2025/150925

(57) **Abstract**

An injector for an implant for an eye disease includes: a cannula comprising an inner space in which at least a part of the implant for an eye disease comprising a ripcord is to be accommodated, and an implant opening into which the implant for an eye disease is to be inserted; a handle part connected to the cannula and to be held by an operator; and a holder configured to be coupled to the handle part and comprising a receiving part in which the ripcord of the implant for an eye disease is to be accommodated. By the injector for an implant for an eye disease, because a ripcord inserted into a tube of the implant for an eye disease is appropriately fixed by being placed in the injector, problems such as the ripcord falling out of the tube, the implant being separated from the injector due to the ripcord, or the ripcord obstructing a surgeon's view during implant surgery may be prevented in advance.

## Description

### [Technical Field]

Embodiments relate to an injector for an implant for an eye disease, and more particularly, to an implant injector for lowering intraocular pressure by draining aqueous humor through a tube-shaped implant inserted into an eyeball, which may easily insert the implant and may minimize damage to the eyeball by fixing a ripcord (stent) inserted into a tube of the implant.

### [Background Art]

For a glaucoma patient whose intraocular pressure is not controlled even by using an intraocular pressure lowering agent, intraocular pressure is lowered by creating a bypass to drain aqueous humor from an anterior chamber of an eye to an external surface of the eye under the conjunctiva. Trabeculectomy among glaucoma filtration surgeries that create a bypass or a fistula for aqueous humor drainage may fail to control intraocular pressure when the amount of aqueous humor drainage decreases due to closure of the bypass after surgery. When initial surgery fails and glaucoma filtration surgery is performed again, the frequency of bypass closure after surgery increases and a success rate of surgery is low.

Also, even in the case of intractable glaucoma such as neovascular glaucoma or secondary glaucoma caused by uveitis according to types of glaucoma, closure of a bypass frequently occurs after trabeculectomy, resulting in poor results. For an eye with a history of failed glaucoma filtration surgery or intractable glaucoma, glaucoma implant surgery of locating a glaucoma implant device is performed to prevent closure of a bypass and increase a success rate of surgery. To date, glaucoma implant surgery has been used as an alternative to trabeculectomy, especially in several difficult-to-treat glaucoma, in that glaucoma implant surgery not only effectively lowers intraocular pressure but also shows a predictable postoperative clinical course according to an inner diameter of a given tube.

However, an existing glaucoma implant used for glaucoma implant surgery may cause various problems and complications such as difficulty in surgery due to a relatively large size, postoperative exposure, infection, eye movement disorder due to a large body, and diplopia. Accordingly, minimally-invasive glaucoma surgery (MIGS) using small-sized glaucoma implant instruments has recently been developed to relatively easily lower intraocular pressure by using a glaucoma implant and to reduce side effects after surgery due to a large size.

Surgery using a small-sized glaucoma implant has an advantage in that surgery may be completed by simply inserting a small-sized glaucoma implant under the conjunctiva into an anterior chamber of an eyeball. However, in order for aqueous humor to be drained from the eyeball at an appropriate pressure, a glaucoma implant with a very small size should be inserted and fixed at an appropriate position within the eyeball.

However, in a conventional injector for an implant for an eye disease, the implant itself is completely inserted into an insertion tube of the injector, the injector is inserted into an eyeball, and the implant is pushed to be inserted into the eyeball. This conventional method has a problem in that ocular surface tissue needs to be damaged as much as an outer diameter of the injector, which is larger than an outer diameter of the implant. Also, this conventional method has a problem in that, when the implant has a structure (e.g., a wing or an arm) that protrudes in a lateral direction rather than a longitudinal direction, an inner diameter of the injector should be increased to cover the entire implant, resulting in an increase in a cross-sectional area of an insertion portion of the injector.

Also, the implant for an eye disease described above may often require additional surgery because the implant may not perform a drainage function due to fibrosis, etc. after surgery. The conventional injector surrounding the implant as described above has a problem in that a range of damage to the ocular surface tissue increases and thus the risk of infection from the outside increases. To solve these problems, there is a need for an insertion method and device for minimizing the range of damage to the ocular surface tissue during implant insertion.

Also, in an implant device for an eye disease, a ripcord for controlling the amount of aqueous humor drainage may be inserted into a tube of the implant device. The ripcord is to be removed when a certain period of time (e.g., one to two months) elapses after implant surgery. However, when the ripcord is not appropriately fixed during the implant surgery, the ripcord may fall out of the implant or the implant may be separated from the injector, and the ripcord may obstruct a surgeon's view.

### [Disclosure]

### [Technical Problem]

To solve the problems described above, the present disclosure may provide an injector for an implant for an eye disease, which may minimize a range of damage to ocular tissue by forming an implant opening in a cannula of the injector for the implant for an eye disease and inserting the implant into an eyeball while fixing a portion of the implant through the implant opening, and may prevent discomfort caused by a ripcord during surgery by appropriately fixing the ripcord inserted into an implant tube.

### [Technical Solution]

An injector for an implant for an eye disease according to an embodiment of the present disclosure comprises: a cannula comprising an inner space in which at least a part of the implant for an eye disease comprising a ripcord is to be accommodated, and an implant opening into which the implant for an eye disease is to be inserted; a handle part connected to the cannula and to be held by an operator; and a holder configured to be coupled to the handle part and comprising a receiving part in which the ripcord of the implant for an eye disease is to be accommodated.

According to an embodiment, the cannula comprises: a first portion comprising one end of the cannula; a second portion connected to the first portion; and a third portion connected to the second portion and comprising the implant opening formed on a side surface.

According to an embodiment, the injector for the implant for an eye disease further comprises the implant for an eye disease. Herein, a body of the implant for an eye disease has a tube shape, one end of the ripcord is inserted through the tube-shaped body, and the other end of the ripcord is inserted into the receiving part of the holder, and at least a portion of the implant for an eye disease is located inside the second portion.

According to an embodiment, the implant for an eye disease comprises a wing portion protruding in a direction different from a longitudinal direction of the implant.

According to an embodiment, the injector for the implant for an eye disease further comprises a cannula assembly coupled to the cannula and configured to be inserted into the holder. Herein, the holder comprises a body having an inner space into which the cannula assembly is to be inserted and through which the cannula coupled to the cannula assembly is to be exposed to outside of the holder.

According to an embodiment, the handle part comprises a protrusion formed at one end of the handle part and at least partially inserted into the holder to couple the handle part to the holder.

According to an embodiment, the protrusion comprises: a first protrusion inserted into the cannula assembly to couple the handle part to the cannula assembly; and a second protrusion connected to the first protrusion, having a larger diameter than the first protrusion, and configured to be inserted into the holder.

According to an embodiment, the holder comprises a projection formed on an inner surface of the holder. Herein, the protrusion comprises a third protrusion connected to the second protrusion, having a larger diameter than the second protrusion, and comprising a coupling protrusion to be engaged with the projection of the holder.

According to an embodiment, the holder further comprises an opening part connected to the inner space of the holder and formed at one end of the holder. Herein, the receiving part penetrates the body of the holder and is connected to the opening part, and the third protrusion comprises a fixing protrusion located at one end of the receiving part when the handle part is coupled to the holder to form a receiving space for the ripcord.

According to an embodiment, the opening part comprises an insertion groove formed at a portion connected to the receiving part and protruding outward from a cross-section of the opening part, and the handle part is configured to be coupled to the holder so that the fixing protrusion is inserted into the insertion groove to fix a coupling angle of the holder.

### [Advantageous Effects]

According to an injector for an implant for an eye disease according to an embodiment of the present disclosure, because a ripcord inserted into a tube of the implant for an eye disease is appropriately fixed by being placed in the injector, problems such as the ripcord falling out of the tube, the implant being separated from the injector due to the ripcord, or the ripcord obstructing a surgeon's view during implant surgery may be prevented in advance.

Also, according to the injector for the implant for an eye disease according to an embodiment of the present disclosure, a range of damage to ocular tissue when the implant for an eye disease is inserted into an anterior chamber of an eyeball may be minimized, and because a portion of the implant not inserted into the anterior chamber is not inserted into the injector but is located outside the injector, an internal structure of the injector may be kept small.

Also, because implant insertion is performed in a state where only a portion of the implant is inserted into the injector, even when a structure such as a wing, an arm, or a sheet that protrudes in a lateral direction is provided at a rear end of the implant, an insertion method or a structure of the injector may be used.

### [Description of Drawings]

FIGS. 1 and 2 illustrate an injector for an implant for an eye disease according to an embodiment of the present disclosure.
FIG. 3 illustrates a cannula 100 according to an embodiment of the present disclosure.
FIG. 4 is side cross-sectional views illustrating a first portion 110 of the cannula 100 according to various embodiments of the present disclosure.
FIG. 5 is a side cross-sectional view illustrating a cannula according to an embodiment of the present disclosure.
FIG. 6 is a view for describing a range of a circumference value of a cannula according to an embodiment of the present disclosure.
FIG. 7A is a perspective view illustrating an injector for an implant for an eye disease according to an embodiment of the present disclosure.
FIG. 7B is a side view illustrating a holder 300 of the injector for an implant for an eye disease of FIG. 7A.
FIG. 8 illustrates coupling of an injector for an implant for an eye disease and the implant according to an embodiment of the present disclosure.
FIGS. 9A to 9C illustrate an implant insertion process of an injector for an implant for an eye disease according to an embodiment of the present disclosure.
FIGS. 9D and 9E illustrate examples of an implant for an eye disease according to an embodiment of the present disclosure.
FIG. 10A is a horizontal cross-sectional view illustrating coupling using a cannula assembly 150 in an injector for an implant for an eye disease according to an embodiment of the present disclosure.
FIG. 10B is a vertical cross-sectional view illustrating coupling using the cannula assembly 150 in the injector for the implant for an eye disease according to an embodiment of the present disclosure.
FIG. 11 illustrates a portion of a handle part 200 coupled to a holder in an implant for an eye disease according to an embodiment of the present disclosure.
FIGS. 12A and 12B illustrate a portion of a holder 300 coupled to a handle part in an implant for an eye disease according to an embodiment of the present disclosure.
FIG. 13 illustrates coupling of the handle part 200 and the holder 300 in an implant for an eye disease according to an embodiment of the present disclosure.
FIG. 14 is a cross-sectional view illustrating a state where an injector for an implant for an eye disease is coupled according to an embodiment of the present disclosure.

### [Mode for Invention]

The terms used herein will be briefly described, and the present disclosure will be described in detail.

The terms used herein are general terms currently widely used in the art in consideration of functions in the present disclosure, but the terms may vary according to the intention of one of ordinary skill in the art, precedents, or new technology in the art. Also, some of the terms used herein may be arbitrarily chosen by the present applicant, and in this case, these terms are defined in detail below. Accordingly, the specific terms used herein should be defined based on the unique meanings thereof and the whole context of the present disclosure.

It will be understood that when a certain part "includes" a certain component, the part does not exclude another component but may further include another component, unless the context clearly dictates otherwise. Also, throughout the specification, when an element is referred to as being "connected" to another element, it will be understood to include that the element is "directly connected" to the other element or is "connected" to the other element with another element therebetween.

The present disclosure will now be described more fully with reference to the accompanying drawings for one of ordinary skill in the art to be able to perform the present disclosure without any difficulty. However, the present disclosure may be embodied in many different forms and is not limited to the embodiments set forth herein. For clarity, portions irrelevant to the descriptions of the present disclosure are omitted in the drawings, and like components are denoted by like reference numerals throughout the specification.

An injector for an implant for an eye disease according to embodiments of the present disclosure is a device for inserting a tube-shaped implant into an eyeball to control intraocular pressure by controlling the drainage amount of aqueous humor generated in an anterior chamber located in front of a crystalline lens in the eyeball. The implant prevents damage to an optic nerve due to increased intraocular pressure caused by eye diseases. The implant for an eye disease according to embodiments of the present disclosure may be used to treat or alleviate symptoms of various eye diseases that cause or are caused by increased intraocular pressure.

Eye diseases in the specification may include glaucoma caused by an increase in intraocular pressure. Examples of glaucoma may include, but are not limited to, congenital glaucoma, traumatic glaucoma, glaucoma suspect, ocular hypertension, primary open-angle glaucoma, normal-tension glaucoma, capsular glaucoma with pseudoexfoliation of lens, chronic simple glaucoma, low-tension glaucoma, pigmentary glaucoma, primary angle-closure glaucoma, acute angle-closure glaucoma, chronic angle-closure glaucoma, intermittent angle-closure glaucoma, glaucoma secondary to eye trauma, glaucoma secondary to eye inflammation, glaucoma secondary to drugs, neovascular glaucoma, and secondary glaucoma due to uveitis.

The implant for an eye disease may have a tube shape applicable to minimally-invasive glaucoma surgery (MIGS), and one end of the tube may be inserted into the anterior chamber of the eyeball and the other end of the tube is inserted into conjunctival tissue or Tenon's tissue. In various embodiments of the present disclosure, the implant may have any of various shapes such as a straight shape, a wedge shape, a cross shape, or a cover shape.

The implant for an eye disease may be inserted after an operator exfoliates the conjunctival tissue or Tenon's tissue of the eyeball, and after insertion, the implant may be placed in the eyeball by covering the conjunctival tissue or Tenon's tissue again. Alternatively, the implant for an eye disease may be partially inserted into the anterior chamber of the eyeball by being inserted into the eyeball through the injector according to an embodiment of the present disclosure. The injector may be a device for injecting the implant into the eyeball by manually or mechanically pushing the implant accommodated therein.

Hereinafter, the present disclosure will be described in detail with reference to the accompanying drawings.

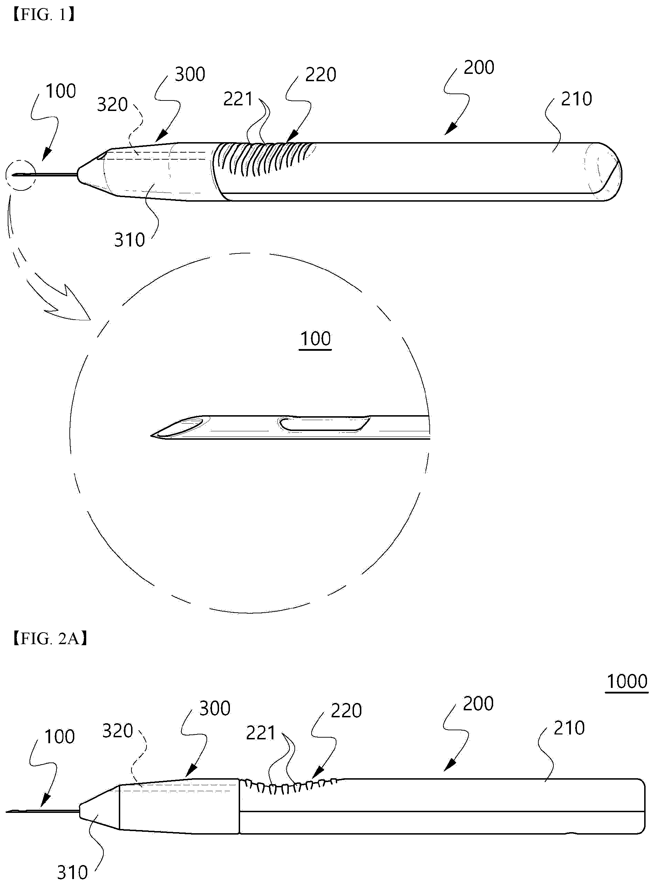

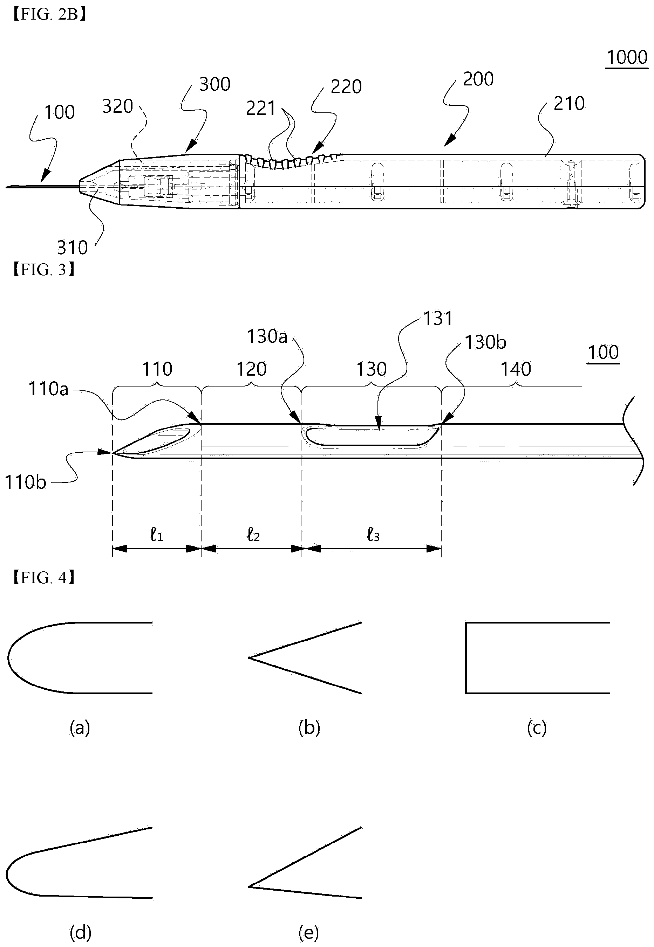

FIGS. 1 and 2 illustrate an injector for an implant for an eye disease according to an embodiment of the present disclosure. Referring to FIG. 1, the injector for the implant for an eye disease may include a cannula 100 having an inner space, a handle part 200 connected to the cannula 100 and held by an operator, and a holder 300 coupled to the handle part 200 and including a receiving part 320 in which a ripcord of the implant for an eye disease is accommodated.

The handle part 200 may include a body 210 and a handle groove 220 for easy holding by the operator. In an embodiment, the handle groove 220 may have a shape in which a part of a surface of the body 210 is recessed inward and one or more protrusions 221 are formed inside the recessed portion to increase a frictional force with the operator's fingers. However, a shape of the handle groove 220 is not limited thereto.

Although not shown, in an example, the handle part 200 may further include a gear structure (not shown) for moving the cannula 100 in a longitudinal direction or rotating the cannula 100. The gear structure may enable delicate cannula control by allowing the cannula 100 to move or rotate in a smaller amount than the operator's hand movement (e.g., forward/backward movement or rotation of a lever). The linear or rotational movement described above may be simultaneously performed through a gear having a spiral structure.

The holder 300 includes a body 310 coupled to the handle part 220, and a receiving part 320 into which a ripcord of the implant for an eye disease is inserted is formed inside the body 310. The receiving part 320 has a hole or channel shape passing through the body 310, and is configured so that the ripcord is inserted and placed from a front end (i.e., a distal end facing a patient) of the holder 300 to a rear end (i.e., a proximal end facing the operator) to be placed, thereby preventing problems such as the ripcord falling out of the implant, the implant being separated from the injector due to the ripcord, or the ripcord obstructing the operator's view during implant surgery.

FIG. 3 illustrates the cannula 100 according to an embodiment of the present disclosure. Referring to FIG. 3, the cannula 100 includes a first portion 110 including one end 110b of the cannula, a second portion 120 connected to the first portion 110, and a third portion 130 connected to the second portion 120 and including an implant opening 131 formed on a side surface. For example, when an end of the cannula has an inclined shape, the first portion refers to a length portion including an inclined portion. Accordingly, when the end of the cannula has a nearly right-angled surface, the first portion may be defined as a length as small as a length of the corresponding surface. That is, when the end of the cannula has an inclined surface, the first portion is defined as an interval l₁ between a first end 110a and a second end 110b of the inclined surface along a longitudinal axis. Accordingly, when the first end 110a and the second end 110b are located on the same vertical line, the first portion may exist a line that does not have a volume.

Also, the third portion may be defined as a length portion in which the implant opening 131 is formed. Also, the second portion may be defined as a portion between the first portion and the third portion.

In an example, a length of the second portion may be 1 mm or more. In the injector according to an embodiment of the present disclosure, an implant is inserted into an eye with only a portion inserted into the cannula. In this case, in order for the implant to be appropriately fixed to the cannula, the implant needs to be inserted by a certain length into the cannula and fit inside the cannula. In this case, the portion of the implant may be located in the second portion inside the cannula. Accordingly, it is preferable that a length of the second portion is at least 1 mm. The implant should be inserted into the cannula by about 1 mm to generate an appropriate fixing force so that the implant does not fall out of the cannula due to intraocular tissue even when the canula is inserted into the eye. A length of the second portion is preferably 1 mm or more, but may be 3 mm or more.

When the canula is inserted into the eye in order to insert the implant, if an insertion depth is too much, there is a risk of damaging the intraocular tissue by touching tissue opposite to the insertion portion. In order to prevent such damage, it is preferable that a sum of lengths of the first portion and the second portion of the cannula has a certain length. In an example, a sum of lengths of the first portion and the second portion may be 10 mm or less. When the sum is greater than 10 mm, there is a risk of damage to the intraocular tissue during a process of inserting the implant and separating the implant in the eye.

That is, when a length of the second portion is too short, there is a high possibility that the implant inserted into the cannula will fall out of the cannula due to the resistance of ocular tissue when the cannula is inserted into the eyeball, and thus, it is preferable that a length of the second portion is 1 mm or more. When a sum of lengths of the first portion and the second portion is greater than 10 mm, the implant inserted into the cannula may not be easily separated from the implant after the cannula is inserted into the eyeball, and damage to the intraocular tissue may occur.

Also, the cannula 100 may further include a fourth portion 140 connected to the third portion 130 and connected to the handle part 200. The fourth portion 140 may be integrally formed with the handle part 200 described above or may be fastened as a separate member to the handle part 200.

FIG. 4 is side cross-sectional views viewed from a side of the first portion 110 of the cannula 100 according to various embodiments of the present disclosure. Referring to FIG. 4, an end of the first portion 110 of the cannula may have, but is not limited to a curved shape (a), a pointed shape (b), a right-angled cross-sectional shape (c), a curved shape inclined to one side (d), or a pointed shape inclined to one side (e). (b) of FIG. 4 may mean that the first portion 110 has a cone shape or a shape in which an end of a cylinder is cut diagonally around one point. That is, in an embodiment of the present disclosure, one end of the first portion 110 of the cannula may have one or more inclined surfaces. Also, in an example, one end of the cannula may be open. Referring to FIGS. 1 to 3, an end of the first portion 110 of the cannula is open. When one end of the cannula is open, an operator may check the implant by protruding the implant through this opening, and may pull the implant out again or may push the implant into the cannula. In this case, because the protruding implant is pushed to an opening surface or a position adjacent to the opening surface, the operator may use the opening at the one end of the cannula as a means for checking and fixing a position of the implant in the cannula.

Referring back to FIG. 3, in the present disclosure, the first portion 110 of the cannula may refer to a section where an inclined surface is formed, the third portion 130 may refer to a section where the implant opening 131 is formed, and the second portion 120 may refer to a section between the first portion 120 and the third portion 130.

Although a side surface of the second portion 120 is closed in FIG. 3, in another embodiment, at least one opening may be formed. However, a longitudinal width of the at least one opening formed in the second portion 120 may be less than a longitudinal width l₃ of the implant opening 131 of the third portion 130. That is, the implant opening 131 is formed at a certain distance from one end of the cannula.

That is, when a length of the second portion is too short, there is a high possibility that the implant inserted into the cannula will fall out of the cannula due to the resistance of the ocular tissue when the cannula is inserted into the eyeball, and thus, it is preferable that a length of the second portion is 1 mm or more. When a sum of lengths of the first portion and the second portion is greater than 10 mm, the implant inserted into the cannula may not be not easily separated from the implant after the cannula is inserted into the eyeball.

In order to solve these problems, in the injector according to an embodiment of the present disclosure, because a sum of lengths of the first portion and the second portion is defined to be 10 mm or less, when the cannula into which the implant is partially inserted is inserted into the eyeball, the implant is maintained in the inserted state, and when the implant is separated from the eyeball, the implant is easily separated.

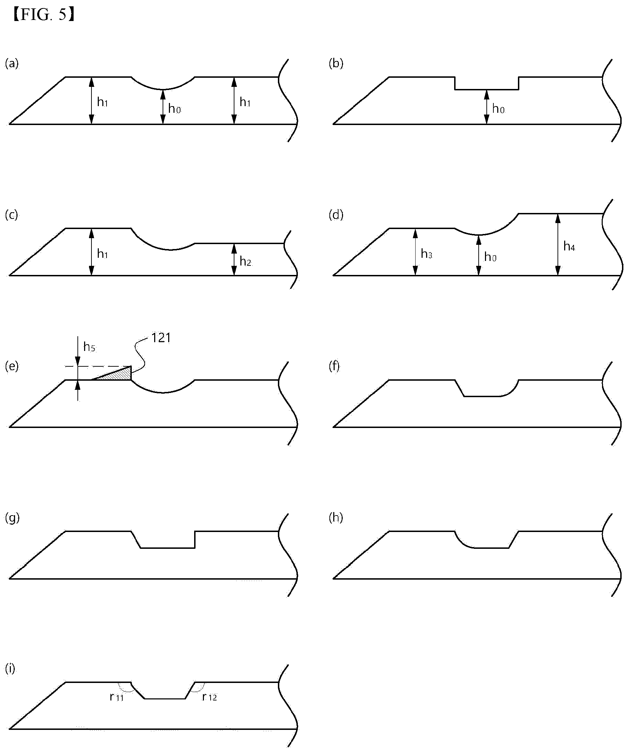

In an example, the implant opening may be defined by a first opening end adjacent to the second portion and a second opening end adjacent to the fourth portion in the longitudinal direction. In this case, heights of the first opening end and the second opening end may be different from each other. In an example, a height of the first opening end may be greater than a height of the second opening end. Hereinafter, a change in heights of both ends of the implant opening in the longitudinal direction and a function thereof will be described with reference to FIG. 5.

FIG. 5 is a side cross-sectional view illustrating a cannula according to an embodiment of the present disclosure. Referring to (a) and (b) of FIG. 5, a heights h₁ of the second portion and the fourth portion may be the same, and a height h₀ of the third portion may be less than the heights h₁ of the second portion and the fourth portion. That is, an implant opening may be formed in a concave shape.

Also, as shown in (c) of FIG. 5, the height h₁ of the second portion may be greater than a height h₂ of the fourth portion. In this case, an angle between an implant inserted into the cannula and the cannula may be less than that in (a) and (b) of FIG. 5. Also, because a height of the second portion is greater than a height of the fourth portion, resistance applied to the implant when the cannula is inserted into an eyeball in a state where the implant is partially inserted into the cannula may be reduced. That is, this is because the implant is inserted while tilted more toward the cannula and thus an insertion area is reduced.

In another embodiment, contrary to (c) of FIG. 5, a height of the fourth portion may be greater than a height of the second portion as shown in (d) of FIG. 5. In this case, an angle between the implant and the cannula may increase. That is, a portion of the implant that is not inserted into the cannula may have a posture that rises above the cannula. In this case, an operator may easily hold the exposed portion of the implant with forceps and may insert the cannula. Alternatively, even when the portion of the implant is not held with forceps, a bent part of the exposed portion of the implant may be pushed and supported by the fourth portion to prevent the implant from falling out when the cannula is inserted.

Also, in still another embodiment, as shown in (e) of FIG. 5, the second portion may include a protrusion 121 located adjacent to the implant opening. The protrusion may have a certain height h₅, and may reduce a force of ocular tissue pushing the exposed portion of the implant when the cannula is inserted into the eyeball. To this end, the protrusion may have a structure whose height gradually increases from the first portion toward the third portion.

Also, in still another embodiment, referring to (f) to (i) of FIG. 5, shapes of both ends of the implant opening 131 in the longitudinal direction may vary such as inclined plane shapes, inclined curved shapes, and shapes with different degrees of inclination. By changing shapes of both ends of the implant opening 131 in the longitudinal direction in this way, an appropriate shape may be adopted for insertion into the cannula, fixing force enhancement during insertion into the eyeball, and easy separation within the eyeball according to a shape of the implant.

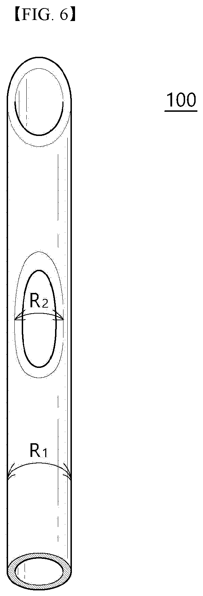

FIG. 6 is a view for describing a range of a circumference value of a cannula according to an embodiment of the present disclosure. Referring to FIG. 6, a circumference R₂ of an implant opening in a second direction (i.e., a rotational direction about the longitudinal direction) may be equal to or less than 40% of a total circumference R₁ of the third portion. That is, it may be advantageous if 100_{*}R₂/R₁ is less than 40. When the value R₂ is too large, there is a risk that a portion of the third portion 130 excluding the implant opening 131 is destroyed or easily damaged by the resistance of intraocular tissue during cannula insertion. Accordingly, in order to prevent such a problem, it is preferable that a value of a circumference of the implant opening does not exceed 40% of the total circumference.

In still another example, a depth of the implant opening may be 40% of an outer diameter of the cannula. As in the example below, a vertical depth of the implant opening (i.e., a height of an empty portion) may be 40% of the outer diameter of the cannula.

### <Example>

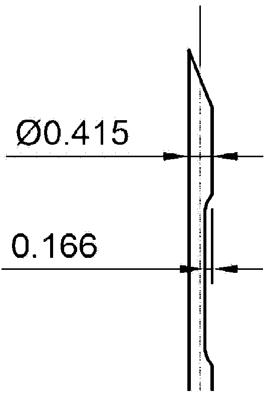

However, this is only an example, and the above ratio may vary according to a material of the cannula. The purpose of describing a ratio of the opening in the specification with reference to FIG. 6 is to explain that the area of the implant opening applied to the cannula should be appropriately adjusted according to a material and a structure of the implant and a material and a structure of the cannula.

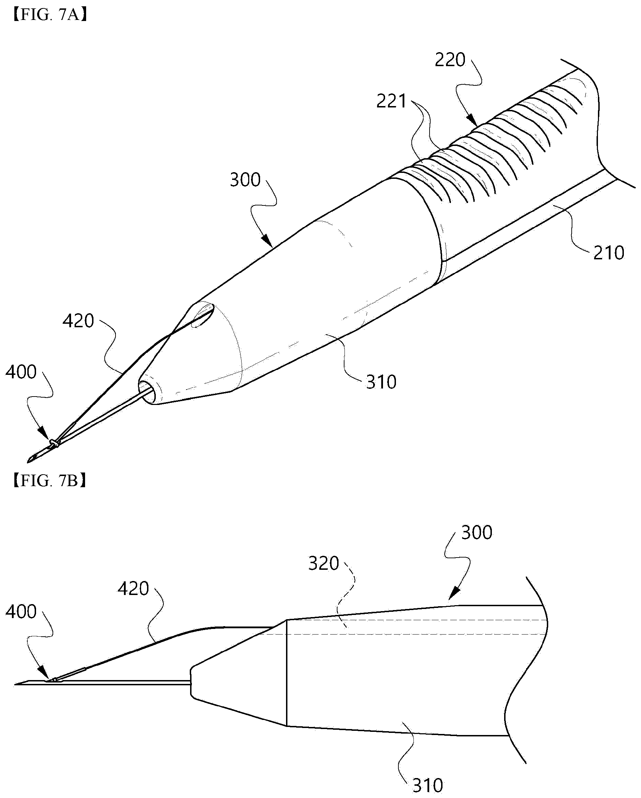

FIG. 7A is a perspective view illustrating an injector for an implant for an eye disease, in which an implant 400 for an eye disease is placed according to an embodiment of the present disclosure. FIG. 7B is a side view illustrating a front end of the holder 300 in the injector for an implant for an eye disease of FIG. 7A. An operator may prepare the injector as shown in FIGS. 7A and 7B, may insert an implant for an eye disease into the injector, and then may perform insertion into an eyeball. Alternatively, the implant may be manufactured and packaged with a part of the implant inserted into the injector and delivered to the operator.

As shown, in the present embodiment, the implant 400 for an eye disease has a tube-shaped body, and includes a ripcord 420, a part of which is inserted into the implant 400 to control the amount of aqueous humor drainage from an eye through the implant 400. Another part of the ripcord 420 is inserted and placed in the receiving part 320 of the holder 300 so that a proximal end of the ripcord 420 facing the operator is fixed while the implant 400 is inserted into a patient's eye by using the injector for the implant for an eye disease. For example, both ends of the ripcord 420 may be respectively inserted into the implant 400 and the holder 300. The end of the ripcord 420 inserted into the implant 400 may completely pass through the implant 400 and may be exposed through a distal end of the implant 400 facing the patient's eye.

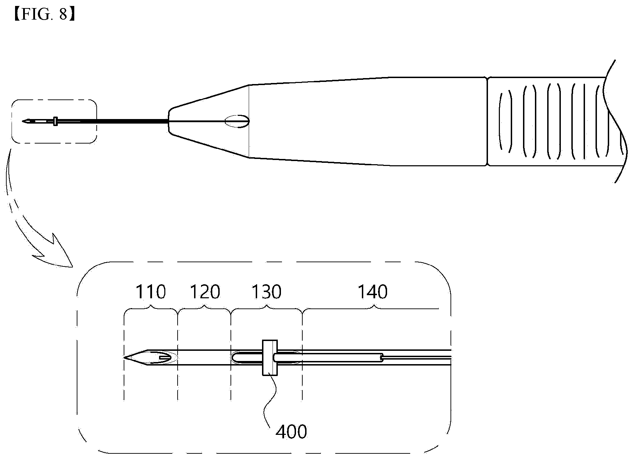

FIG. 8 illustrates an injector for an implant for an eye disease according to an embodiment of the present disclosure. Referring to FIG. 8, the injector for an implant for an eye disease may further include an implant 300 for an eye disease. The implant for an eye disease may have a tube shape, and at least a portion of the implant for an eye disease may be located inside the second portion of the cannula. In order for one portion of the implant to be located inside the second portion of the cannula, the implant may be inserted toward one end through the implant opening 131 or inserted into one end so that a portion protrudes to the outside through the implant opening 131.

In an example, the implant 300 for an eye disease may have a tube shape extending in the longitudinal direction and having an inner diameter.

In an embodiment, the implant may be formed of, but not limited to, silicone, PTFE, polycarbonate, polyurethane, polyethylene, polypropylene, polyimide, poly(methyl methacrylate) (PMMA), poly(styrene-b-isobutylene-b-styrene), polyethersulfone, gelatin, stainless steel, titanium, nitinol, or a combination thereof.

In an embodiment, the implant may be formed in a curved shape with a certain curvature in order to prevent damage to the endothelium of a cornea in an eyeball. A front end of the implant may poke and damage the cornea in an anterior chamber of the eyeball in a process of inserting the implant into the anterior chamber of the eyeball according to a size of the eyeball that is different for each patient, a skill level in implant injection, etc. Damage to the cornea may cause complications such as corneal decompensation which require even future corneal transplantation.

In an embodiment, in order for the implant to naturally move in a curved shape while being inserted into the anterior chamber of the eyeball, the implant may be manufactured in a curved shape with a certain curvature corresponding to a curvature of a surface of the eyeball. Alternatively, even a straight implant with no curvature may be used by using a material with sufficient elasticity or flexibility. Also, although a portion of the implant not inserted into the cannula includes a part extending in a direction different from the longitudinal direction, this is only an example, and the implant may extend only in one direction or may have any of other arbitrary structures.

Referring to FIG. 8, only one portion of the implant 300 for an eye disease passes through the implant opening and is inserted into the cannula toward the first portion (one end). That is, in the present disclosure, the implant 300 for an eye disease is only partially inserted into the cannula of the injector and is not completely inserted into the injector or inserted into the eyeball or inserted into the eyeball with the injector inserted into the implant. When the implant for an eye disease is inserted into the injector, the injector is inserted into the eyeball, and the implant is removed from the injector, a range of movement of the injector within the eyeball to separate the injector and the implant may increase, thereby increasing a range of damage to ocular tissue. Also, a structure of the injector may be complicated to implement a separate mechanism for separating the implant inserted into the injector, thereby increasing manufacturing costs. Also, it may be difficult to apply a method of inserting the injector into the implant to a micrometer-sized implant member such as MIGS.

In the injector for the implant for an eye disease according to an embodiment of the present disclosure, because only at least a portion of the implant 300 is inserted into the cannula and then the cannula is inserted into the eyeball, in a process of inserting the cannula into the eyeball, 1) a range of damage to ocular surface tissue may be limited only to a portion of a cross-sectional thickness of the cannula + a cross-sectional thickness of the implant, and 2) the implant and the cannula may be simply separated in the eyeball.

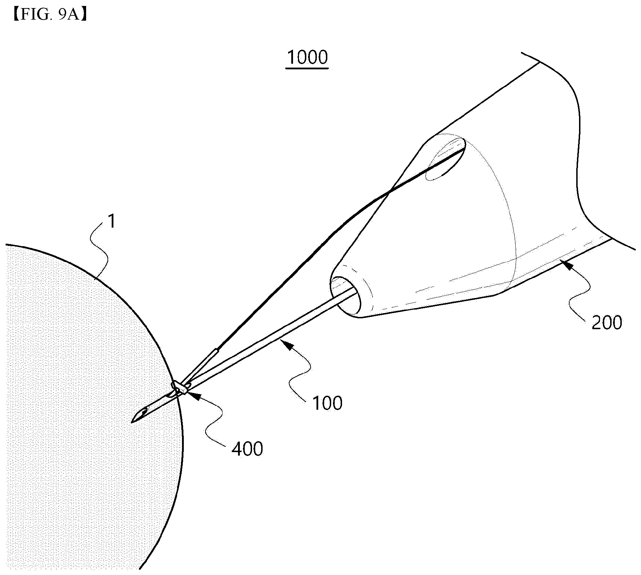

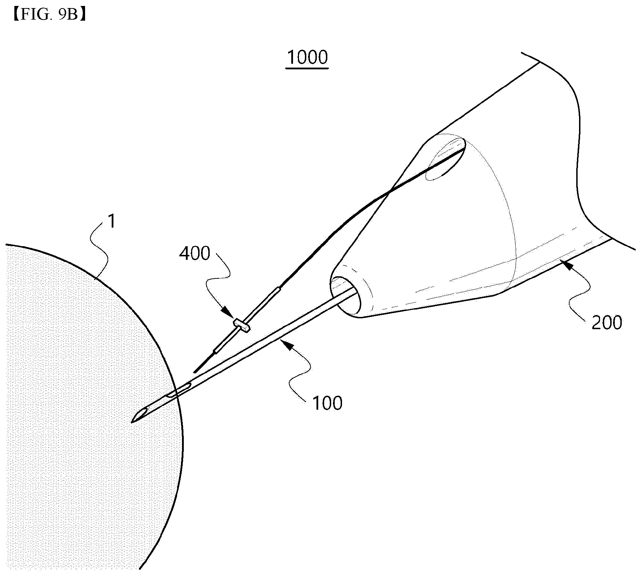

FIGS. 9A to 9C illustrate an implant insertion process of an injector for an implant for an eye disease according to an embodiment of the present disclosure. Referring to FIG. 9A, an operator may insert a cannula into an eyeball 1 in a state where a portion of the implant 400 is inserted through an implant opening as shown in FIG. 8. The operator may open a conjunctiva around a corneal limbus with surgical scissors or may create a scleral flap according to a condition of a sclera. Here, the scleral flap may have a substantially quadrangular or trapezoidal shape with a size of about 3 mm in width and length and may have a depth of about ±50% of a thickness of the sclera, but the present disclosure is not limited thereto.

The operator may insert the cannula into the eyeball in a state where the implant is inserted (loaded) into the cannula. For example, the operator may push the cannula to a position at least 1 to 2 mm away from the corneal limbus. In this case, the implant inserted into an anterior chamber may be located as far away from a cornea as possible.

As the cannula 100 is inserted into the eyeball 1, the implant 300 inserted into the cannula may also be inserted into the eyeball. In this case, a surface of the eyeball may be opened by a cross-sectional area of a non-inserted portion of the implant and a cross-sectional area of the cannula. The implant 400 is formed of a very flexible material, and thus, as the cannula is inserted, the implant receives resistance from intraocular tissue and adheres to a side surface of the cannula. Accordingly, the area of the surface of the eyeball that is opened may be minimized.

In an example, when the implant 400 is located at a required position in the eyeball 1, the operator needs to fix the implant at that position and remove the cannula 100 from the eyeball.

To this end, the operator may separate the implant from the cannula by rotating one end of the cannula 100 in a certain direction (opposite to a direction in which the implant opening faces). That is, the operator may separate the implant through the implant opening by rotating the cannula 100 in a pitch direction. Alternatively, the operator may easily separate the implant by simultaneously rotating the cannula in the pitch direction and moving the cannula forward by a certain distance. For example, it is preferable that the cannula is moved forward by about 1 mm, but the present disclosure is not limited thereto.

This separation is possible because the first portion and the second portion of the cannula are located in the eye chamber and the fourth portion is located on or outside the surface of the eyeball, and thus, the fourth portion is supported by ocular surface tissue (the surface tissue tightens the cannula and the implant), and when the operator rotates the cannula in the pitch direction based on this support, the implant located in the eye chamber may be easily separated from the implant opening.

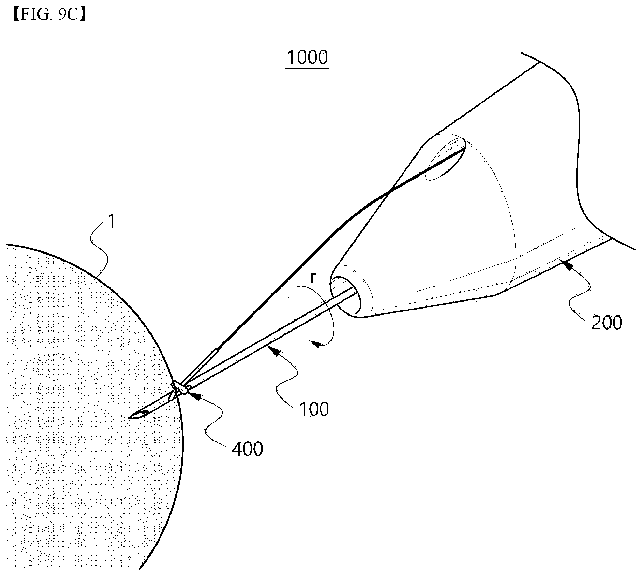

In another example, when the implant in the eyeball reaches a certain position, the operator may separate the implant from the cannula by rotating the cannula in a roll direction, rather than rotating the cannula in the pitch direction. Referring to FIG. 9C, the implant is separated from the implant opening when the operator rotates the cannula 100 in a roll direction r. When rotating the cannula in the roll direction, the operator may perform roll rotation while moving the cannula forward by a certain length (about 1 mm), similar to pitch rotation described above. When this forward movement is accompanied, the implant may be more easily separated. Also, in the case of roll direction rather than pitch rotation, because the cannula is rotated as it is, a range of the already opened ocular surface tissue may not be additionally expanded compared to the case of pitch rotation.

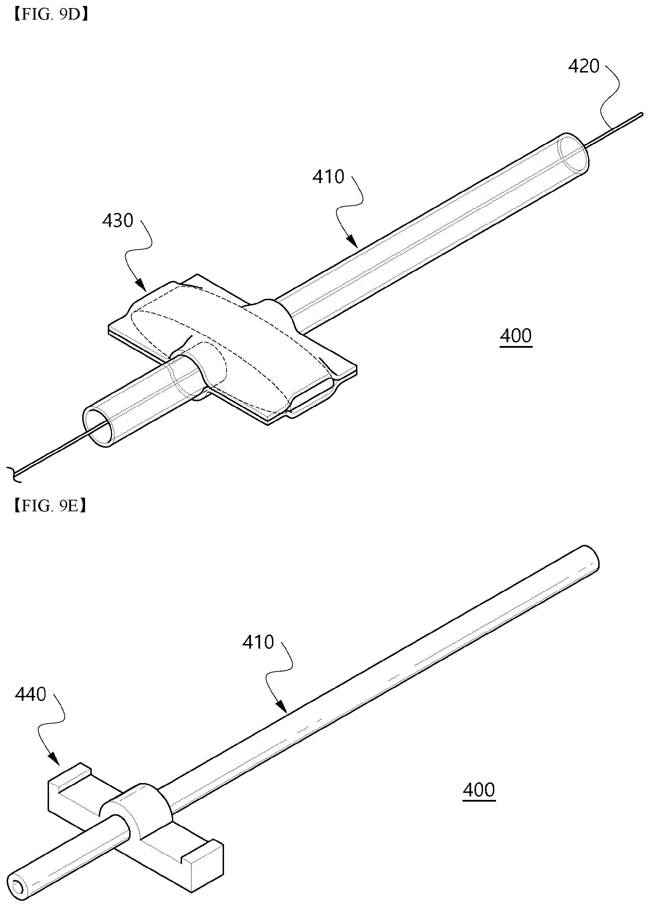

FIGS. 9D and 9E illustrate examples of the implant 400 for an eye disease according to an embodiment of the present disclosure. In an example, the implant for an eye disease may include a body 410 and a wing portion 440 protruding in a direction other than a longitudinal direction of the body 410. The wing portion 440 is illustrated in FIGS. 9A to 9D. The wing portion 440 may be perpendicular to the longitudinal direction of the body or may be inclined at a certain angle. Also, the wing portion 440 may extend in one direction, but may have a part that additionally protrudes in another direction from a side (see FIG. 9E). The wing portion may prevent the implant inserted into an eyeball from entering the eyeball.

For example, the implant 400 for an eye disease may include the ripcord 420 inside the body. The ripcord 420 may prevent aqueous humor from rapidly leaking immediately after the implant is inserted.

Also, referring to FIG. 9D, the implant 400 for an eye disease may further include a matrix covering at least partially covering the implant. The matrix 430 may perform a function that is the same as or similar to that of the wing portion. That is, the matrix 430 may prevent the implant from entering the eyeball. Also, the matrix 430 may be loaded with an eye disease drug. For example, the eye disease drug may be immersed in the matrix, or injected into an inner space of the matrix and then may be gradually discharged in an eye.

In an embodiment, examples of the drug to be loaded into the matrix may include, but are not limited to, dexamethasone, mitomycin-C (MMC), 5-fluorouracil (5-FU), triamcinolone (TA), anti-vascular endothelial growth factor (anti-VEGF) drug, and transforming growth factor (TGF)-beta inhibitor. For example, according to a purpose of applying a drug to the matrix, in addition to the antifibrotic agent, an intraocular pressure lowering agent such as prostaglandin, a Cephalosporin-based or another type of antibiotic such as levofloxacin, moxifloxacin, or ceftazidime, or another type of drug may be loaded into the matrix. Also, the matrix may be formed of a material that does not immediately decompose in a living body, allowing sufficient time for the drug to be released, and does not swell excessively enough to cause foreign body sensation in the eye due to the drug. In an embodiment, a polymer having a membrane structure capable of forming a storage space for capturing the drug may be used as the matrix.

For example, the matrix 430 may be formed of, but is not limited to, poly(acrylic acid), polyacrylamide, poly(sulfopropyl acrylate), poly(2-hydroxyethyl methacrylate), poly(vinyl alcohol), silicone, polyurethane, collagen, gelatin, hyaluronic acid, poly(aspartic acid), alginate, hydroxypropyl cellulose, hydroxypropyl methylcellulose acetate succinate, chitosan, or a combination thereof.

In an embodiment, the matrix 430 may be a material having a double-layer structure formed of two or more different materials. In this case, a first material of the matrix 430 may be a material having biocompatibility and mechanical properties for forming the skeleton of the matrix 430 without being immediately biodegraded in the eyeball. Also, a second material, which is another material constituting the matrix 430 together with the first material, may be a material that has hydrophilicity and may temporarily absorb a water-soluble drug when combined with the first material. When the matrix 430 includes a composite of the first material and the second material as in the present embodiment, a drug suitable for the purpose may be easily impregnated in the matrix 430 while maintaining the mechanical strength of the matrix 430. For example, the matrix 430 may be formed as a sheet including a composite of PU and PEG.

In an embodiment, the first material may be, but is not limited to, silicone, polyethylene vinyl acetate, polyvinyl acetate, polycarbonate, polyvinyl chloride, polyurethane, PMMA, poly(butyl methacrylate), polyamide, polyethylene, polypropylene, polyethylene terephthalate (PET), glycol-modified PTE, PTFE, polyhydroxyalkanoates, parylene, polyether ether ketone, polyimide, epoxy-based resin such as SU-8, poly(vinylidene fluoride), polyether block amides, 3-[tris(trimethylsiloxy)silyl]propyl methacrylate, poly(styrene-b-isobutylene-b-styrene) copolymer, cellulose acetate, poly((N-vinylpyrrolidone)-block-poly(vinyl acetate)), ethyl cellulose, or a combination thereof.

Also, in an embodiment, the second material may be, but is not limited to, poly(ethylene glycol) (PEG), PEG derivatives, poly(ethylene oxide), poly(propylene oxide), polyvinyl alcohol, polyvinyl pyrrolidone, polyethersulfone, polyamide, polyacrylamide, polyglycolic acid, polyacrylic acid, glucuronic acid, hexuronic acid, hyaluronic acid, polyaspartic acid, alginate, polyorthoester, 2-hydroxyethyl methacrylate, collagen, gelatin, hydroxypropyl cellulose, chitosan, or a combination thereof.

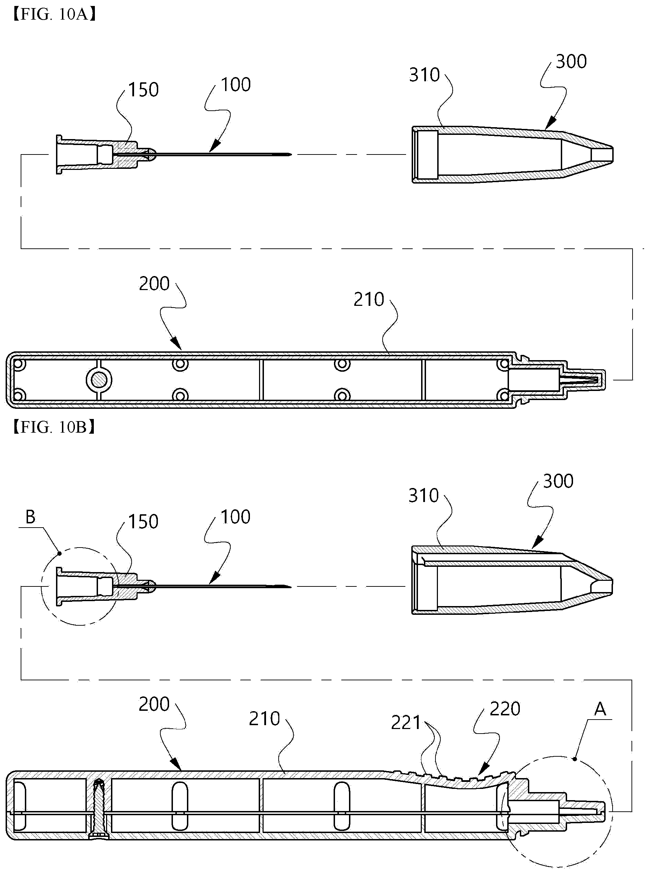

FIG. 10A is a horizontal cross-sectional view illustrating coupling using a cannula assembly 150 in an injector for an implant for an eye disease according to an embodiment of the present disclosure. FIG. 10B is a vertical cross-sectional view illustrating coupling using the cannula assembly 150 in the injector for the implant for an eye disease according to an embodiment of the present disclosure.

Referring to FIGS. 10A and 10B, the injector for the implant for an eye disease further includes the cannula assembly 150 coupled to the cannula 100 and inserted into the holder 300. The cannula assembly 150 may include a hole or a channel having a diameter almost similar to a diameter of the cannula 100, and may be coupled to the cannula 100 by inserting one end of the cannula 100 into the hole or the channel.

Also, an opening is formed at a rear end of the cannula assembly 150, that is, at a proximal end facing an operator when the operator holds the injector for the implant for an eye disease, and the handle part 200 may be inserted into the opening. The injector for the implant for an eye disease according to the present embodiment may be prepared by coupling a rear end portion B of the cannula assembly 150 coupled to the cannula 100 to a front end portion A of the handle part 200 (a distal end facing a patient's eyeball) and coupling the holder 300 to the handle part 200 to cover the front end portion A of the handle part 200 and the cannula assembly 150.

Because an inner space of the holder 300 completely passes through the body 310 of the holder 300, in a state where the holder 300 is coupled to the cannula assembly 150, an end of the cannula 100 coupled to the cannula assembly 150 may be exposed toward the patient's eyeball through a front end (i.e., a distal end) of the holder 300.

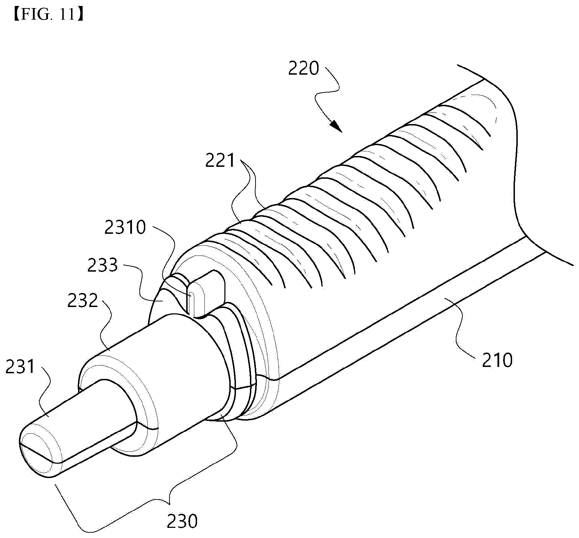

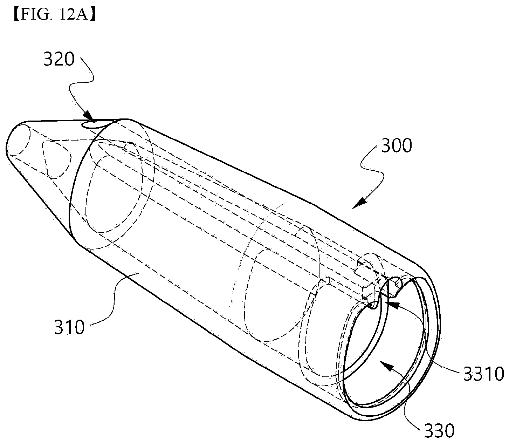

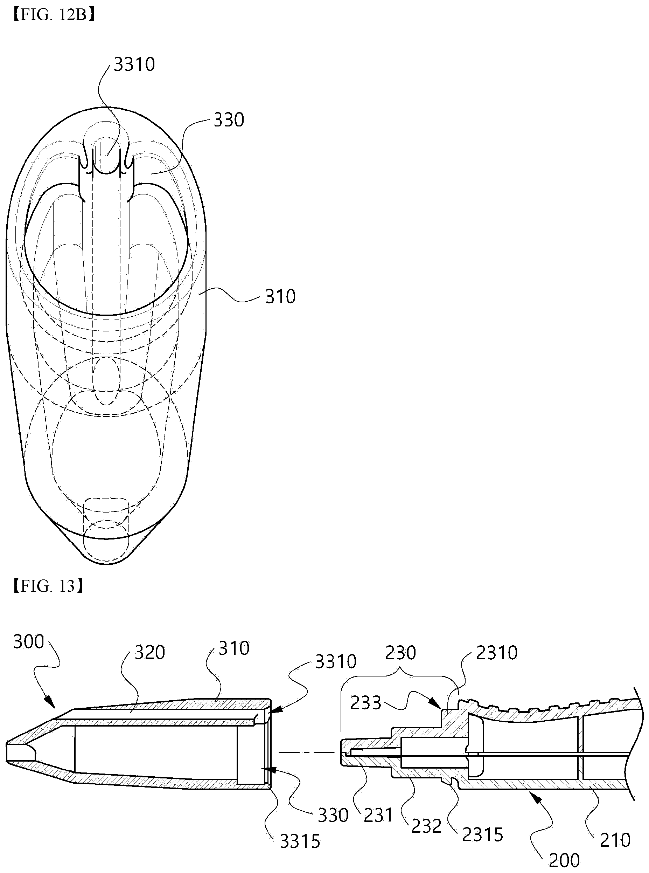

FIG. 11 is a perspective view illustrating a coupling portion of the handle part 200 coupled to a holder in an injector for an implant for an eye disease according to an embodiment. FIGS. 12A and 12B are perspective views illustrating a coupling portion of the holder 300 coupled to a handle part in an injector for an implant for an eye disease according to an embodiment.

Referring to FIG. 11, the handle part 200 may include a protrusion 230 to be inserted into the holder 300 to couple the handle part 200 to the holder 300. The protrusion 230 may be formed at one end of the body 210 of the handle part 200, and the handle part 200 may be coupled to the holder 300 by inserting at least a part of the protrusion 230 into an inner space of the holder 300. Accordingly, the protrusion 230 may be located at a front end (e.g., a distal end facing a patient's eyeball) of the handle part 200.

In an embodiment, the protrusion 230 of the handle part 200 may include one or more steps having different diameters. That is, the protrusion 230 may include a first protrusion 231 located at a front end of the protrusion 230 and inserted into the cannula assembly 150 to couple the handle part 200 and the canula assembly 150 to each other, and a second protrusion 232 connected to the first protrusion 231, located at a rear end of the first protrusion 231, and having a larger dimeter than the first protrusion 231. The cannula assembly 150 may be coupled to the handle part 200 by being fitted to the first protrusion 231, and an edge end of the cannula assembly 150 may be located on the second protrusion 232.

The second protrusion 232 may have a diameter sufficient to be inserted into the inner space of the holder 300, and the holder 300 may be coupled to the handle part 200 by inserting the second protrusion 232 into the holder 300.

In an embodiment, the protrusion 230 of the handle part 200 may further include a third protrusion 233 located at a rear end of the second protrusion 232 and having a larger diameter than the second protrusion 232. That is, because the third protrusion 233 is formed at one end of the body 210 of the handle part 200, the second protrusion 232 having a smaller diameter than the third protrusion 233 is formed on the third protrusion 233, and the first protrusion 231 having a smaller diameter than the second protrusion 232 is formed on the second protrusion 232, a multi-step protrusion structure in which diameters of protrusions decrease away from the body 210 may be formed.

Referring to FIGS. 12A and 12B, the holder 300 includes the receiving part 320 formed in a hole or a channel shape penetrating from a front end to a rear end of the body 310 of the holder 300. However, this is only an example, and in another embodiment, the receiving part 320 may be formed only in a certain portion of the body 310. Also, the holder 300 has an inner space in which the cannula assembly 150 and the protrusion 230 of the handle part 200 are inserted and coupled to each other, and an opening part 330 connected to the inner space is formed at one end of the body 310 of the holder 300. The holder 300 and the handle part 200 may be coupled to each other by inserting the cannula assembly 150 and a portion of the handle part 200 coupled to the cannula assembly 150 through the opening part 330.

In an embodiment, a cross-section of the opening part 330 may have a circular, elliptical, polygonal, or any other closed curved shape, and a portion of the opening part 330 may protrude outward from the cross-section to form an insertion groove 3310. The third protrusion 233 of the handle part 200 described with reference to FIG. 11 may include a fixing protrusion 2310 formed at a position corresponding to the insertion groove 3310 of the holder 300 and protruding outward from a cross-section of the third protrusion 233.

When the holder 300 and the handle part 200 are fixed by inserting the fixing protrusion 2310 of the third protrusion 233 into the insertion groove 3310 of the holder 300, the fixing protrusion 230 and the insertion groove 2230 protruding and extending in one direction unlike other portions of both members may be engaged with each other, thereby preventing a coupling angle between the handle part 200 and the holder 300 from changing (e.g., the holder 300 from rotating). Accordingly, because a coupling angle of the holder 300 to the handle part 200 is stably fixed during surgery of inserting the implant for an eye disease, a ripcord of the implant for an eye disease placed in the receiving part 320 of the holder 300 is prevented from moving.

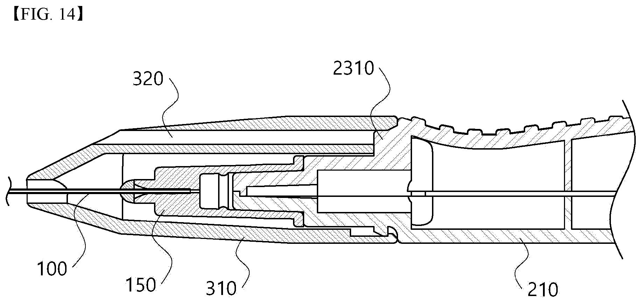

FIG. 13 is a vertical cross-sectional view illustrating coupling of the handle part 200 and the holder 300 in an implant for an eye disease according to an embodiment of the present disclosure. FIG. 14 is a vertical cross-sectional view illustrating a state where an injector for an implant for an eye disease is coupled according to an embodiment of the present disclosure. A horizontal cross-section may be easily understood from coupling through a vertical cross-section, and thus, a detailed description thereof will be omitted.

Referring to FIGS. 13 and 14, the receiving part 320 of the holder 300 may be formed to penetrate the body 310 of the holder 300, and when the holder 300 is coupled to the handle part 200, the third protrusion 233 of the handle part 200 may be located at an end of the receiving part 320 to block the end of the receiving part 320 and form a receiving space for a ripcord. For example, the fixing protrusion 2310 of the third protrusion 233 for preventing rotation of the holder 300 relative to the handle part 200 may be inserted into the insertion groove 3310 of the holder 300 and located at the end of the receiving part 320.

A projection 3315 protruding toward an inner space of the holder 300 is formed on a portion of an inner surface of the holder 300, and a coupling protrusion 2315 is formed on the protrusion 230 of the handle part 200 at a position corresponding to the projection 3315. As shown in FIG. 14, when the holder 300 is coupled to the handle part 200, the projection 3315 and the coupling protrusion 2315 may be engaged with each other so that the holder 300 is prevented from being separated from the handle part 200 unless a large force greater than a certain level is applied.

Although cross-sections are shown in FIGS. 13 and 14, each of the projection 3315 and the coupling protrusion 2315 may extend along an edge of each member. For example, the remaining portion except for the fixing protrusion 2310 at an edge of the third protrusion 233 of the handle part 200 may correspond to the coupling protrusion 2315 coupled to the projection 2315.

The above description of the present disclosure is provided for illustration, and it will be understood by one of ordinary skill in the art that various changes in form and details may be readily made therein without departing from essential features and the scope of the present disclosure. Accordingly, the above embodiments are examples only in all aspects and are not limited. For example, each component described as a single type may be executed in a distributed manner, and components described as a distributed type may be executed in a combined manner.

The scope of the present disclosure is indicated by the claims rather than by the detailed description of the present disclosure, and it should be understood that the claims and all modifications or modified forms drawn from the concept and scope of the claims and equivalents are included in the scope of the present disclosure.

### [Industrial Applicability]

Embodiments relate to an injector for an implant for an eye disease, and more particularly, to an implant injector for lowering intraocular pressure by draining aqueous humor through a tube-shaped implant inserted into an eyeball, which may easily insert the implant and may minimize damage to the eyeball by fixing a ripcord inserted into a tube of the implant.

## Claims

1. An injector for an implant for an eye disease, comprising:
a cannula comprising an inner space in which at least a part of the implant for an eye disease comprising a ripcord is to be accommodated, and an implant opening into which the implant for an eye disease is to be inserted;
a handle part connected to the cannula and to be held by an operator; and
a holder configured to be coupled to the handle part and comprising a receiving part in which the ripcord of the implant for an eye disease is to be accommodated.

2. The injector for the implant for an eye disease according to claim 1, wherein the cannula comprises:
a first portion comprising one end of the cannula;
a second portion connected to the first portion; and
a third portion connected to the second portion and comprising the implant opening formed on a side surface.

3. The injector for the implant for an eye disease according to claim 2, further comprising the implant for an eye disease,
wherein a body of the implant for an eye disease has a tube shape,
one end of the ripcord is inserted through the tube-shaped body, and the other end of the ripcord is inserted into the receiving part of the holder, and
at least a portion of the implant for an eye disease is located inside the second portion.

4. The injector for the implant for an eye disease according to claim 3, wherein the implant for an eye disease comprises a wing portion protruding in a direction different from a longitudinal direction of the implant.

5. The injector for the implant for an eye disease according to claim 1, further comprising a cannula assembly coupled to the cannula and configured to be inserted into the holder,
wherein the holder comprises a body having an inner space into which the cannula assembly is to be inserted and through which the cannula coupled to the cannula assembly is to be exposed to outside of the holder.

6. The injector for the implant for an eye disease according to claim 5, wherein the handle part comprises a protrusion formed at one end of the handle part and at least partially inserted into the holder to couple the handle part to the holder.

7. The injector for the implant for an eye disease according to claim 6, wherein the protrusion comprises:
a first protrusion inserted into the cannula assembly to couple the handle part to the cannula assembly; and
a second protrusion connected to the first protrusion, having a larger diameter than the first protrusion, and configured to be inserted into the holder.

8. The injector for the implant for an eye disease according to claim 7, wherein the holder comprises a projection formed on an inner surface of the holder,
wherein the protrusion comprises a third protrusion connected to the second protrusion, having a larger diameter than the second protrusion, and comprising a coupling protrusion to be engaged with the projection of the holder.

9. The injector for the implant for an eye disease according to claim 8, wherein the holder further comprises an opening part connected to the inner space of the holder and formed at one end of the holder,
wherein the receiving part penetrates the body of the holder and is connected to the opening part, and
the third protrusion comprises a fixing protrusion located at one end of the receiving part when the handle part is coupled to the holder to form a receiving space for the ripcord.

10. The injector for the implant for an eye disease according to claim 9, wherein
the opening part comprises an insertion groove formed at a portion connected to the receiving part and protruding outward from a cross-section of the opening part, and
the handle part is configured to be coupled to the holder so that the fixing protrusion is inserted into the insertion groove to fix a coupling angle of the holder.
